# EUROPÄISCHE PATENTSCHRIFT

(11) **EP 2 653 123 B1**
(45) Veröffentlichungstag und Bekanntmachung des Hinweises auf die Patenterteilung: **01.06.2016**
(21) Anmeldenummer: 13002038.1
(22) Anmeldetag: 18.04.2013
(51) Int. Cl.: A61B 17/295, A61B 18/14

(54) **Werkzeug für ein medizinisches Instrument**
Tool for a medical instrument
Outil pour un instrument médical

(30) Priorität: 18.04.2012 DE 102012007652
(43) Veröffentlichungstag der Anmeldung: 23.10.2013
(73) Patentinhaber: Karl Storz GmbH & Co. KG, 78532 Tuttlingen (DE)
(72) Erfinder: Merz, Robin, 78532 Tuttlingen (DE); Stefan, Jochen, 78532 Tuttlingen (DE)

(56) Entgegenhaltungen:
- EP-A1- 2 316 367
- US-A- 6 024 741
- US-A1- 2002 099 372
- US-A1- 2006 226 195
- US-A1- 2011 060 333

## Beschreibung

Die vorliegende Erfindung ist auf ein Werkzeug für ein medizinisches Instrument und auf ein Verfahren zum Zerlegen eines Werkzeugs bezogen.

Die Erwartungen an medizinische Instrumente, insbesondere an medizinische Instrumente für mikroinvasive Eingriffe steigen beständig. Bereits in Vielfalt angeboten und weit verbreitet sind medizinische Instrumente mit einem Werkzeug mit Greif- oder Schneidefunktion am distalen Ende. Medizinische Einrichtungen und medizinisches Personal bevorzugen möglichst vielfältig verwendbare medizinische Instrumente. Je vielfältiger das einzelne medizinische Instrument einsetzbar ist, desto geringer ist die Vielfalt der bereitzuhaltenden Instrumente. Vielfältig einsetzbare medizinische Instrumente können deshalb auch bei höheren Stückkosten die in einer medizinischen Einrichtung erforderlichen Investitionen senken. Durch eine geringe Vielfalt und eine geringere Stückzahl der bereitzuhaltenden medizinischen Instrumente können ferner die Kosten für Lagerhaltung und Logistik verringert werden. Bei mikroinvasiven medizinischen Instrumenten bzw. medizinischen Instrumenten für mikroinvasive Eingriffe kommt hinzu, dass während eines Eingriffs umso seltener Instrumente gewechselt werden müssen, je vielfältiger das einzelne Instrument einsetzbar ist.

Aus der Veröffentlichung US 2011/0060333 ist ein Werkzeug für ein medizinisches gemäß dem Oberbegriff von Anspruch 1 bekannt.

Eine Aufgabe der vorliegenden Erfindung besteht darin, ein verbessertes Werkzeug für ein medizinisches Instrument, ein verbessertes medizinisches Instrument und ein verbessertes Verfahren zum Zerlegen eines Werkzeugs für ein medizinisches Instrument zu schaffen.

Diese Aufgabe wird durch die Gegenstände der unabhängigen Ansprüche gelöst.

Weiterbildungen sind in den abhängigen Ansprüchen angegeben.

Ein Werkzeug für ein medizinisches Instrument umfasst eine Wirkeinrichtung, eine Übertragungsstange, deren distales Ende mit der Wirkeinrichtung gekoppelt ist, zum Übertragung zumindest entweder einer Kraft oder eines Drehmoments zu der Wirkeinrichtung, und eine Schneideeinrichtung, die zum Schneiden von Gewebe in einem Kanal in der Übertragungsstange und in der Wirkeinrichtung bewegbar ist.

Das Werkzeug ist insbesondere für ein mikroinvasiv-chirurgisches Instrument vorgesehen und ausgebildet. Die Wirkeinrichtung ist insbesondere eine Zange mit mehreren Maulteilen, von denen mindestens eines bewegbar ist, zum Greifen oder Quetschen von Gewebe. Alternativ oder zusätzlich kann die Wirkeinrichtung eine elektrochirurgische Funktion aufweisen. In der Elektrochirurgie bzw. bei elektrochirurgischen Maßnahmen wird durch Stromfluss und aufgrund des elektrischen Widerstands von Gewebe (Joulesche) Wärme im Gewebe erzeugt. Durch Form und Anordnung von dabei verwendeten Elektroden wird der Stromfluss möglichst genau lokalisiert. Durch die entstehende Wärme wird das stromdurchflossene Gewebe verödet bzw. zerstört. Dadurch können Gewebe beispielsweise verklebt oder verschlossen und Blutungen gestillt werden. In der Regel werden in der Elektrochirurgie hochfrequente Wechselströme verwendet, um eine Stimulation von Nerven und andere unerwünschte Nebenwirkungen zu vermeiden. Die Begriffe "Elektro-chirurgie" und "HF-Chirurgie" werden deshalb oft synonym verwendet. Ein weiterer, oft synonym verwendeter Begriff ist der der Elektrokauterisation.

Die Wirkeinrichtung und die Schneideeinrichtung sind zwei nicht identische Einrichtungen, die insbesondere ausgebildet sind, um auf unterschiedliche Weisen auf Gewebe einzuwirken und dazu zumindest teilweise unterschiedliche Merkmale und Bestandteile aufweisen. Auch wenn die Wirkeinrichtung beispielsweise eine Greifeinrichtung ist, die ausgebildet ist, um Gewebe während eines Schneidens mittels der Schneideeinrichtung zu halten, weisen die Wirkeinrichtung und die Schneideeinrichtung zwei einander potenziell ergänzende, jedoch verschiedene Funktionen auf. Insbesondere weisen die Schneideeinrichtung und die Wirkeinrichtung keine gemeinsamen Bestandteile auf. Die Schneideeinrichtung kann als vollständig eigenständiges und von der Wirkeinrichtung zerstörungsfrei trennbares Bauteil ausgebildet sein. Die Wirkeinrichtung ist insbesondere ausgebildet, um ihre vorgesehene Funktion und Wirkung auch in Abwesenheit der Schneideeinrichtung aufzuweisen bzw. ausüben zu können.

Das Werkzeug ist insbesondere zur lösbaren mechanischen Verbindung mit einem distalen Ende eines Außenschafts ausgebildet. Die Übertragungsstange ist insbesondere zur Anordnung in dem Außenschaft ausgebildet. Die Übertragungsstange kann gerade oder gekrümmt, starr oder flexibel sein. Wenn das Werkzeug dafür vorgesehen und ausgebildet ist, mit einem gekrümmten oder flexiblen Außenschaft zu einem medizinischen Instrument zusammengesetzt zu werden, ist die Übertragungsstange insbesondere zumindest in gekrümmten oder krümmbaren Bereichen des Außenschafts biegeflexibel oder biegeelastisch. Das proximale Ende der Übertragungsstange ist insbesondere zur Kopplung mit einer Handhabungseinrichtung, insbesondere mit einem Hebel oder einer anderen Betätigungseinrichtung an einer Handhabungseinrichtung ausgebildet.

Der Kanal, in dem die Schneideeinrichtung bewegbar ist, erstreckt sich insbesondere in Längsrichtung des Werkzeugs. Die Längsrichtung des Werkzeugs ist insbesondere identisch mit der Längsrichtung eines distalen Endes eines Außenschafts, wenn das Werkzeug mit dem Außenschaft verbunden ist. Der Kanal erstreckt sich insbesondere im Wesentlichen bis zum distalen Ende der Wirkeinrichtung. Beispielsweise im Falle einer als Greifeinrichtung mit zwei Maulteilen ausgebildeten Wirkeinrichtung erstreckt sich der Kanal vorzugsweise über den größten Teil (insbesondere mindestens 80%) der Länge der Maulteile.

Ein Werkzeug mit den beschriebenen Merkmalen kann besonders vielfältig verwendbar sein. Beispielsweise kann es sowohl zum Greifen, Präparieren und Koagulieren von Gewebe mittels der Wirkeinrichtung als auch zum nachfolgenden Durchtrennen des Gewebes mittels der Schneideeinrichtung verwendbar sein. Dies ermöglicht ein Ausführen komplexer medizinischer Eingriffe ohne einen Wechsel des medizinischen Instruments oder mit weniger häufigem Wechsel des medizinischen Instruments bei einem Eingriff.

Bei einem Werkzeug, wie es hier beschrieben ist, ist die Übertragungsstange insbesondere mit einem schwenkbaren Maulteil des Werkzeugs gekoppelt.

Insbesondere ist das distale der Übertragungsstange mit einem, zwei oder mehr schwenkbaren Maulteilen des Werkzeugs derart gekoppelt, dass eine Bewegung der Übertragungsstange in deren Längsrichtung bzw. in Längsrichtung eines Außenschafts, mit dem das Werkzeug mechanisch verbunden ist, mit einem Schwenken des oder der schwenkbaren Maulteile um Schwenkachsen senkrecht zu der Längsachse einhergeht.

Bei einem Werkzeug, wie es hier beschrieben ist, weist die Wirkeinrichtung insbesondere zwei oder mehr voneinander elektrisch isolierte Maulteile auf, wobei ein erstes der Maulteile mit der Übertragungsstange elektrisch leitfähig verbunden ist, und wobei ein zweites der Maulteile mit einem Außenschaft elektrisch leitfähig verbunden oder verbindbar ist.

Mit zwei oder mehr voneinander elektrisch isolierten Maulteilen ist eine Verwendung als bipolares elektrochirurgisches Instrument möglich, bei dem ein hochfrequenter Wechselstrom zum Koagulieren bzw. Veröden von Gewebe fast ausschließlich in dem kleinen und vergleichsweise wohl definierten Raumbereich zwischen den Maulteilen fließt. Damit kann das Werkzeug beispielsweise zunächst zum elektrochirurgischen Verschließen und unmittelbar danach zum Durchtrennen eines Gefäßes oder eines anderen Hohlorgans verwendbar sein.

Bei einem Werkzeug, wie es hier beschrieben ist, ist die Schneideeinrichtung insbesondere ausgebildet, um bei einer Bewegung in Richtung parallel zur Längsachse des Werkzeugs zu schneiden.

Insbesondere weist die Schneideeinrichtung an ihrem distalen Ende eine zumindest teilweise nach distal gerichtete (insbesondere nicht zur Längsachse und zur vorgesehenen Bewegungsrichtung parallele) Schneide auf. Durch Verschieben der Schneideeinrichtung von einer proximalen Position zu einer distalen Position kann Gewebe geschnitten bzw. durchtrennt werden, das beispielsweise mittels der Wirkeinrichtung gehalten wird.

Bei einem Werkzeug, wie es hier beschrieben ist, umfasst der Kanal insbesondere eine Nut in der Übertragungsstange, wobei zumindest entweder die Schneideeinrichtung oder die Nut so ausgebildet sind, dass eine Bewegung der Schneideeinrichtung nach proximal mit einem Herausheben der Schneideeinrichtung aus der Nut einhergeht.

Insbesondere sind die Schneideeinrichtung und die Nut so ausgebildet, dass das proximale Ende der Schneideeinrichtung aus der Nut herausgehoben wird, wenn es sich dem proximalen Ende der Nut annähert oder das proximale Ende der Nut erreicht. Dies kann eine Entnahme der Schneideeinrichtung aus der Nut durch eine einfache Bewegung der Schneideeinrichtung nach proximal ermöglichen.

Bei einem Werkzeug, wie es hier beschrieben ist, umfasst der Kanal insbesondere eine Nut in der Übertragungsstange, wobei die Nut in einem Bereich nahe dem proximalen Ende der Nut eine nach proximal kontinuierlich abnehmende Tiefe aufweist.

Insbesondere steigt der Grund bzw. der Boden der Nut rampenförmig mit einer konstanten oder variierenden Steigung an.

Alternativ oder zusätzlich weist die Schneideeinrichtung an ihrem proximalen Ende einen rampenförmigen Bereich auf, der über den Rand der Nut an deren proximalem Ende gleiten kann, wobei eine Bewegung der Schneideeinrichtung nach proximal mit einem Herausheben der Schneideeinrichtung aus der Nut einhergeht.

Ein Werkzeug, wie es hier beschrieben ist, umfasst insbesondere eine Kupplungseinrichtung zum lösbaren mechanischen Verbinden des Werkzeugs mit einem distalen Ende eines Außenschafts, wobei in allen für die Schneideeinrichtung während der Verwendung des Werkzeugs vorgesehene Positionen das proximale Ende der Schneideeinrichtung proximal der Kupplungseinrichtung und das distale Ende der Schneideeinrichtung distal der Kupplungseinrichtung angeordnet sind.

Die Schneideeinrichtung weist insbesondere eine Kupplungseinrichtung zum lösbaren mechanischen Verbinden der Schneideeinrichtung mit einem distalen Ende eines Innenschafts oder einer Übertragungseinrichtung auf. Die Anordnung des proximalen Endes der Schneideeinrichtung und ihrer Kupplungseinrichtung proximal der zum lösbaren mechanischen Verbinden des Werkzeugs mit einem Außenschaft vorgesehenen Kupplungseinrichtung kann hinsichtlich des erforderlichen und des zur Verfügung stehenden Bauraums und damit hinsichtlich des konstruktiven und fertigungstechnischen Aufwands und des erreichbaren Miniaturisierungsgrads vorteilhaft sein.

Die Schneideeinrichtung ist insbesondere einstückig ausgebildet, beispielsweise aus einem einzigen Stück Metall hergestellt.

Bei einem Werkzeug, wie es hier beschrieben ist, weist die Schneideeinrichtung insbesondere zwischen ihrem proximalen Ende und ihrem distalen Ende einen stabförmigen Bereich auf.

Der stabförmige Bereich der Schneideeinrichtung weist insbesondere einen rechteckigen Querschnitt auf und ist insbesondere ausgebildet, um vollständig innerhalb des Kanals angeordnet zu werden. Der stabförmige Bereich ist insbesondere bei der vorgesehenen Verwendung und Anordnung der Schneideeinrichtung vollständig innerhalb der erwähnten Nut in der Übertragungsstange angeordnet, ragt also nicht oder nicht wesentlich über die Ränder der Nut hinaus.

Bei einem Werkzeug, wie es hier beschrieben ist, weist die Schneideeinrichtung insbesondere einen Vorsprung auf, der in einer Richtung senkrecht zur vorgesehenen Bewegungsrichtung der Schneideeinrichtung vorsteht.

Der Vorsprung ist insbesondere an oder nahe dem proximalen Ende der Schneideeinrichtung angeordnet und definiert insbesondere das proximale Ende des erwähnten stabförmigen Bereichs der Schneideeinrichtung, indem der Vorsprung eine Abweichung von der stabförmigen Gestalt darstellt. Bei einer Anordnung der Schneideeinrichtung in der erwähnten Nut in der Übertragungsstange ragt lediglich der Vorsprung aus der Nut heraus und bildet eine Kupplungseinrichtung zur lösbaren, insbesondere formschlüssigen, mechanischen Verbindung der Schneideeinrichtung mit einem distalen Ende eines Innenschafts oder einer anderen Übertragungseinrichtung. Ferner kann der Vorsprung beim Zerlegen des Werkzeugs dazu dienen, die Schneideeinrichtung manuell nach proximal zu verschieben, um sie, wie oben beschrieben, aus der Nut herauszuheben.

Bei einem Werkzeug, wie es hier beschrieben ist, ist die Schneideeinrichtung insbesondere in einer während der Verwendung des Werkzeugs vorgesehenen Position von dem Vorsprung abgesehen vollständig in dem Kanal angeordnet.

Ein medizinisches Instrument umfasst ein Werkzeug, wie es hier beschrieben ist, und einen Innenschaft, dessen distales Ende mit der Schneideeinrichtung lösbar mechanisch verbindbar ist.

Bei einem medizinischen Instrument, wie es hier beschrieben ist, umfasst der Innenschaft insbesondere an seinem distalen Ende einen L-förmigen Schlitz oder eine L-förmige Nut zur Aufnahme des Vorsprungs an der Schneideeinrichtung.

Der L-förmige Schlitz oder die L-förmige Nut am distalen Ende des Innenschafts einerseits und der Vorsprung an der Schneideeinrichtung andererseits bilden korrespondierende Bajonett-Kupplungseinrichtungen. Damit kann der Innenschaft mit einer axialen und einer nachfolgenden rotatorischen Bewegung relativ zu dem Werkzeug und der Übertragungsstange mit der Schneideeinrichtung gekoppelt bzw. mechanisch verbunden und durch eine umgekehrte Relativbewegung wieder von ihr getrennt werden.

Bei einem Verfahren zum Zerlegen eines Werkzeugs für ein medizinisches Instrument, wobei das Werkzeug eine Wirkeinrichtung und eine Schneideeinrichtung in einem Kanal umfasst, werden eine mechanische Verbindung zwischen einem distalen Ende einer Übertragungseinrichtung und der Schneideeinrichtung getrennt und die Schneideeinrichtung relativ zum Werkzeug nach proximal bewegt, wobei die Schneideeinrichtung aus dem Kanal herausgehoben wird.

Das Verfahren ist insbesondere auf ein Werkzeug mit den hier beschriebenen Merkmalen anwendbar. Die mechanische Verbindung zwischen dem distalen Ende der Übertragungseinrichtung und der Schneideeinrichtung (insbesondere dem proximalen Ende der Schneideeinrichtung) erfolgt zerstörungsfrei bzw. reversibel. Dazu weisen das distale Ende der Übertragungseinrichtung und die Schneideeinrichtung (insbesondere das proximale Ende der Schneideeinrichtung) zueinander korrespondierende Kupplungseinrichtungen auf.

Das zumindest teilweise Herausheben der Schneideeinrichtung aus dem Kanal erfolgt beim Bewegen der Schneideeinrichtung nach proximal automatisch bzw. selbsttätig aufgrund von Merkmalen der Schneideeinrichtung und/oder des Kanals. Beim Herausheben der Schneideeinrichtung aus dem Kanal wird insbesondere das proximale Ende der Schneideeinrichtung aus einer Nut in einer Übertragungsstange herausgehoben. Dabei gleitet insbesondere das proximale Ende der Schneideeinrichtung an einem rampenförmigen Bereich am proximalen Ende der Nut und/oder ein rampenförmiger Bereich am proximalen Ende der Schneideeinrichtung an einem proximalen Rand der Nut.

Zum Bewegen der Schneideeinrichtung kann das proximale Ende der Schneideeinrichtung, insbesondere der erwähnte Vorsprung, manuell oder mittels eines Hilfsmittels gegriffen werden. Beispielsweise wird ein Finger auf den Vorsprung gedrückt und der Vorsprung aufgrund von Form- und/oder Kraftschluss mit einer Bewegung des Fingers nach proximal mitgenommen.

Das hier beschriebene Verfahren zum Zerlegen eines Werkzeugs kann der Nachbereitung einer Verwendung eines medizinischen Instruments mit dem Werkzeug und der Vorbereitung der Reinigung und Sterilisierung des Werkzeugs und damit auch der Vorbereitung einer weiteren Verwendung im Rahmen eines diagnostischen, chirurgischen oder therapeutischen Verfahrens dienen. Das Verfahren zum Zerlegen stellt aber selbst kein diagnostisches, chirurgisches oder therapeutisches Verfahren dar.

Bei einem Verfahren, wie es hier beschrieben ist, wird insbesondere vor dem Trennen der mechanischen Verbindung zwischen dem distalen Ende der Übertragungseinrichtung und der Schneideeinrichtung ein distales Ende eines Außenschafts von dem Werkzeug getrennt.

### Kurzbeschreibung der Figuren

Nachfolgend werden Ausführungsformen anhand der beigefügten Figuren näher erläutert. Es zeigen:
- Figur 1: eine schematische Darstellung eines medizinischen Instruments;
- Figur 2: eine schematische axonometrische Darstellung einer Greifeinrichtung für ein medizinisches Instrument;
- Figur 3: eine schematische axonometrische Darstellung einer Schneideeinrichtung;
- Figur 4: eine weitere schematische axonometrische Darstellung der Einrichtungen aus den Figur 2 und 3;
- Figur 5: eine weitere schematische axonometrische Darstellung der Einrichtungen aus den Figuren 2 bis 4;
- Figur 6: eine schematische Schnittdarstellung des Werkzeugs aus den Figuren 2,4 und 5;
- Figur 7: eine weitere schematische Schnittdarstellung des Werkzeugs aus den Figuren 2 und 4 bis 6;
- Figur 8: eine weitere schematische Schnittdarstellung des Werkzeugs aus den Figuren 2 und 4 bis 7;
- Figur 9: eine weitere schematische Schnittdarstellung des Werkzeugs aus den Figuren 2 und 4 bis 8;
- Figur 10: ein schematisches Flussdiagram eines Verfahrens zum Zerlegen eines Werkzeugs.

### Beschreibung der Ausführungsformen

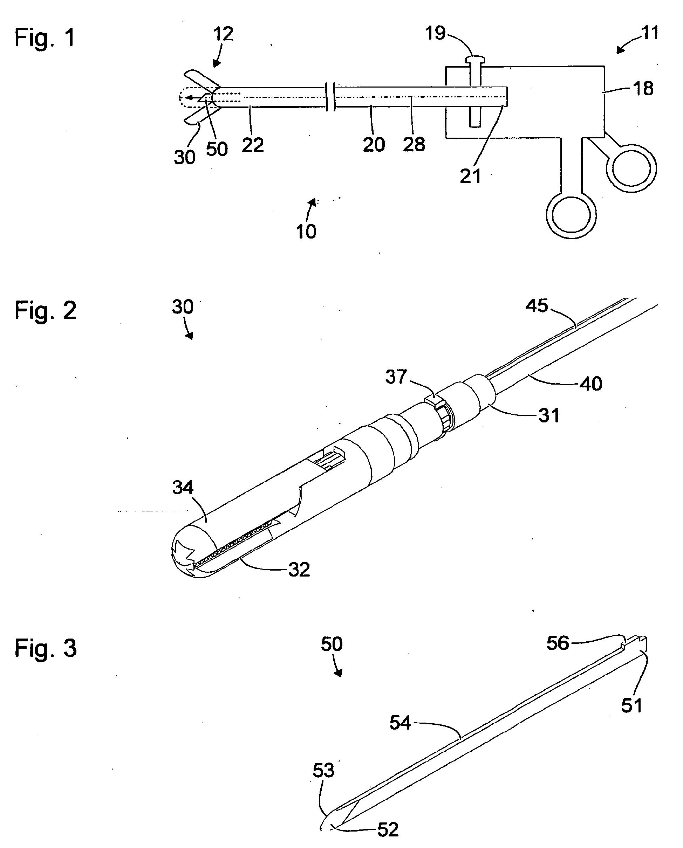

Figur 1 zeigt eine schematische Darstellung eines medizinischen Instruments 10 mit einem proximalen Ende 11 und einem distalen Ende 12. Am proximalen Ende 11 weist das medizinische Instrument 10 eine Handhabungseinrichtung 18 auf, die dazu ausgebildet ist, von medizinischem Personal mit einer Hand gefasst zu werden und manuell erzeugte Kräfte und Momente aufzunehmen. Dazu weist die Handhabungseinrichtung 18 insbesondere mehrere Griffteile auf, die zumindest teilweise relativ zueinander bewegbar sind.

Ein Schaft 20 erstreckt sich vom proximalen Ende 11 bzw. der Handhabungseinrichtung 18 bis zum distalen Ende 12 des medizinischen Instruments 10. Der Schaft 20 weist ein proximales Ende 21 und ein distales Ende 22 auf. Das proximale Ende 21 des Schafts 20 ist mit der Handhabungseinrichtung 18 mechanisch verbunden, insbesondere in einer Ausnehmung mit einer zum proximalen Ende 21 des Schafts 20 korrespondierenden Gestalt angeordnet und dort mittels einer Verriegelungseinrichtung 19 formschlüssig verriegelt.

Der Schaft 20 weist eine Längsachse 28 auf. Im Falle einer kreiszylindrischen Gestalt des Schafts 20 ist die Längsachse 28 insbesondere die Symmetrieachse der Mantelfläche des Schafts 20. Auch der unten beschriebene innere Aufbau des Schafts 20 kann rotationssymmetrisch zur Längsachse 28 sein. Der Schaft 20 kann gerade oder- abweichend von der Darstellung in Figur 1 - gekrümmt, starr oder flexibel sein. Wenn der Schaft 20 zumindest abschnittsweise gekrümmt oder flexibel ist, ist mit der Längsachse nachfolgend die Längsachse des Schafts 20 an seinem proximalen Ende 21 oder an seinem distalen Ende 22 gemeint. Der Schaft 20 ist insbesondere auch im in der Handhabungseinrichtung 18 verriegelten Zustand um seine Längsachse 28 rotierbar.

Das distale Ende 22 des Schafts 20 ist mit einem Werkzeug verbunden, das eine Greifeinrichtung 30 und eine Schneideeinrichtung 50 umfasst. Die Greifeinrichtung 30 weist insbesondere zwei Greifbacken auf, von denen mindestens eine um eine Schwenkachse senkrecht zur Zeichenebene der Figur 1 schwenkbar ist. Bei dem in Figur 1 gezeigten Beispiel sind beide Greifbacken zwischen offenen Positionen, die in Figur 1 mit durchgezogenen Linien dargestellt sind, und geschlossenen Positionen, die in Figur 1 mit gestrichelten Linien dargestellt sind, schwenkbar. Die Schneideeinrichtung 50 ist, wie in Figur 1 durch einen Pfeil angedeutet, in Richtung parallel zur Längsachse 28 bewegbar, und zwar sowohl wenn die Maulteile der Greifeinrichting ihre offenen als auch wenn sie ihre geschlossenen Positionen einnehmen.

Nachfolgend sind Ausführungsbeispiele des Werkzeugs 30, 50 und seiner lösbaren mechanischen Verbindung mit einem Außenschaft, einem Innenschaft und einer Übertragungsstange dargestellt. Die nachfolgend dargestellten Werkzeuge, Außenschäfte, Innenschäfte und Übertragungsstangen können zur Bildung eines medizinischen Instruments mit den anhand der Figur 1 dargestellten Merkmalen und/oder mit anderen Merkmalen ausgebildet sein und verwendet werden.

Figur 2 zeigt eine schematische axonometrische Darstellung einer Greifeinrichtung 30, die vorgesehen und ausgebildet ist zur Bildung eines medizinischen Instruments, wie es oben anhand der Figur 1 dargestellt ist. Die Greifeinrichtung 30 weist ein proximales Ende 31 und zwei Maulteile 32, 34, die das distale Ende der Greifeinrichtung 30 bilden, auf. Im Gegensatz zu der in Figur 1 angedeuteten Greifeinrichtung umfasst die in Figur 2 dargestellte Greifeinrichtung 30 ein feststehendes Maulteil 32 und ein schwenkbares Maulteil 34. Nahe dem proximalen Ende 31 weist die Greifeinrichtung 30 zwei symmetrisch angeordnete Bajonettklauen bzw. Knaggen 37 auf, von denen eine an einer vom Betrachter abgewandten Seite angeordnet und deshalb weitgehend verdeckt ist.

Die Greifeinrichtung 30 ist mit einer Übertragungsstange 40 mechanisch verbunden. Die Übertragungsstange 40 ist relativ zur Greifeinrichtung 30, insbesondere relativ zum proximalen Ende 31 und zum feststehenden Maulteil 32 in axialer Richtung, d.h. parallel zur Längsachse der Übertragungsstange 40 und zur Längsachse 28 des Schafts 20 (vgl. Figur 1) innerhalb eines vorbestimmten Intervalls bewegbar. Das innerhalb der Greifeinrichtung 30 angeordnete und deshalb in Figur 2 nicht sichtbare distale Ende der Übertragungsstange 40 ist mit dem schwenkbaren Maulteil 34 derart gekoppelt, dass eine axiale Bewegung der Übertragungsstange 40 mit einer Schwenkbewegung des schwenkbaren Maulteils 34 einhergeht. In der Übertragungsstange 40 ist eine Nut 45 vorgesehen, die insbesondere einen schmalen und tiefen rechteckigen Querschnitt aufweist. Die Nut 45 in der Übertragungsstange 40 ist an deren in Figur 2 nicht sichtbaren distalen Ende durch einen Kanal entsprechenden Querschnitts fortgesetzt, der sich zwischen den Maulteilen 32, 34 bis fast zu deren distalen Enden erstreckt.

Teile der Greifeinrichtung 30, insbesondere die Knaggen 37 sowie die Übertragungsstange 40 sind aus Edelstahl oder einem anderen Metall gefertigt. Die Knaggen 37 und die Übertragungsstange 40 sind voneinander elektrisch isoliert. Die Maulteile 32, 34 weisen metallische und damit elektrisch leitfähige Greifflächen auf, die voneinander elektrisch isoliert sind, wenn sie nicht, wie in Figur 2 gezeigt, aneinander anliegen. Die Knaggen 37 und die Übertragungsstange 40 sind jeweils mit der Greiffläche eines Maulteils 32, 34 elektrisch leitfähig verbunden. Insbesondere sind die Knaggen 37 mit der Greiffläche des feststehenden Maulteils 32 und die Übertragungsstange 40 mit der Greiffläche des schwenkbaren Maulteils 34 elektrisch leitfähig verbunden.

Figur 3 zeigt eine schematische axonometrische Darstellung einer Schneideeinrichtung 50 mit einem proximalen Ende 51 und einem distalen Ende 52. Am distalen Ende 52 weist die Schneideeinrichtung 50 eine Schneide 53 auf. Am proximalen Ende weist die Schneideeinrichtung 50 einen Vorsprung 56 auf. Zwischen dem proximalen Ende 51 und dem distalen Ende 52 umfasst die Schneideeinrichtung einen stabförmigen Bereich 54, der im Wesentlichen die Gestalt einer streifenförmigen Platte bzw. eines Stabs mit rechteckigem Querschnitt aufweist.

Zwischen dem Vorsprung 56 am proximalen Ende 51 und der Schneide 53 am distalen Ende 52 entspricht der Querschnitt der Schneideeinrichtung 50 im Wesentlichen dem Querschnitt der Nut 45 in der Übertragungsstange 40 (vgl. Figur 2), so dass die Schneideeinrichtung 50 von dem Vorsprung 56 abgesehen, vollständig von der Nut 45 in der Übertragungsstange 40 aufgenommen und in dieser spiel- und reibungsarm geführt und in Längsrichtung der Übertragungsstange 40 und der Schneideeinrichtung 50 verschiebbar sein kann. Der Vorsprung 56 ist vorgesehen, um aus der Nut 45 in der Übertragungsstange 40 herauszuragen.

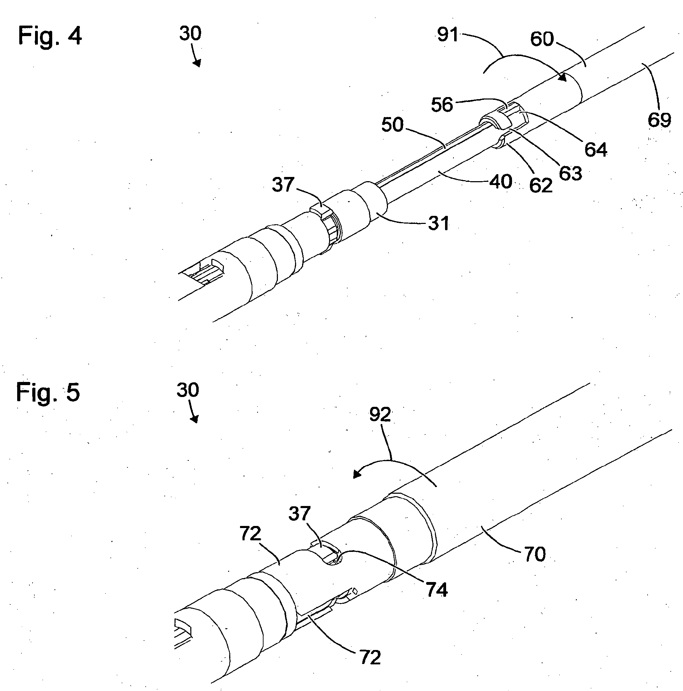

Figur 4 zeigt eine weitere schematische axonometrische Darstellung der Greifeinrichting aus Figur 2 und der Schneideeinrichtung 50 aus Figur 3. Bei der Darstellung in Figur4 ist die Schneideeinrichtung 50 in der Nut 45 in der Übertragungsstange 40 (vgl. Figur 2) angeordnet. Das distale Ende 52 der Schneideeinrichtung 50 (vgl. Figur 3) ist dabei in der Greifeinrichtung und insbesondere zwischen den Maulteilen 32, 34 angeordnet. Der Vorsprung 56 ragt aus der Nut 45 heraus.

In Figur 4 ist ferner ein Innenschaft 60 gezeigt, der im Wesentlichen eine rohrförmige bzw. kreiszylindermantelförmige Gestalt aufweist. Der Innenschaft 60 weist an seinem distalen Ende 62 einen L-förmigen Schlitz mit einem axialen bzw. sich in axialer Richtung erstreckenden Abschnitt 63 und einem umfänglichen bzw. in umfänglicher Richtung sich erstreckenden Abschnitt 64 auf. Die in umfänglicher Richtung zu messende Breite des axialen Abschnitts 63 und die in axialer Richtung zu messende Breite des umfänglichen Abschnitts 64 des L-förmigen Schlitzes sind an die Abmessungen des Vorsprungs 56 an der Schneideeinrichtung 50 angepasst.

Nach einem Einführen der Übertragungsstange 40 in den Innenschaft 60 kann der Vorsprung 56 durch eine Relativbewegung in axialer Richtung durch den axialen Abschnitt 63 bis in den umfänglichen Abschnitt 64 eingeführt werden. Wenn der Vorsprung 56 an der Schneideeinrichtung 50 sich im umfänglichen Abschnitt 64 des L-förmigen Schlitzes am distalen Ende 62 des Innenschafts 60 befindet, kann der Innenschaft 60 relativ zu der Greifeinrichtung 30, der Übertragungsstange 40 und der Schneideeinrichtung 50 in einer ersten Richtung 91 bis zu der in Figur 4 gezeigten Konfiguration rotiert werden.

In der in Figur 4 gezeigten relativen Positionierung von Schneideeinrichtung 50 und Innenschaft 60 sind Schneideeinrichtung 50 und Innenschaft 60 hinsichtlich axialer Bewegungen miteinander (von Spiel abgesehen) starr gekoppelt. Eine axiale Bewegung des Innenschafts 60 geht deshalb mit einer entsprechenden axialen Bewegung der Schneideeinrichtung 50 einher. Somit kann mittels des Innenschafts 60 eine Bewegung der Schneide 53 am distalen Ende 52 der Schneideeinrichtung 50 (vgl. Figur 3) in dem erwähnten, in den Figuren nicht sichtbaren Kanal zwischen den Maulteilen 32, 34 bewirkt werden, um beispielsweise von den Maulteilen 32, 34 begriffenes Gewebe nach einer Elektrokauterisation zu durchtrennen.

Der Innenschaft 60 weist einen Isoliermantel 69 auf, dessen distaler Rand nahe dem L-förmigen Schlitz 63, 64 liegt, und der sich bis nahe des proximalen Endes des Innenschafts 60 erstrecken kann.

Figur 5 zeigt eine weitere schematische axonometrische Darstellung der Greifeinrichtung 30 aus den Figuren 2 und 4. Die Darstellung in Figur 5 unterscheidet sich von der Darstellung in den Figuren 2 und 4 dadurch, dass ein Außenschaft 70 mit der Greifeinrichtung 30 gekoppelt ist. Der Außenschaft 70 weist an seinem distalen Ende 72 zwei symmetrisch angeordnete L-förmige Schlitze mit je einem axialen Abschnitt 73 und einen umfänglichen Abschnitt 74 auf. Einer der L-förmigen Schlitze ist an einer vom Betrachter abgewandten Seite des Außenschafts 70 angeordnet und deshalb in Figur 5 nicht sichtbar.

Die in umfänglicher Richtung zu messende Breite der axialen Abschnitte 73 und die in axialer Richtung zu messende Breite der umfänglichen Abschnitte 74 der L-förmigen Schlitze sind an die Abmessungen der Knaggen 37 an der Greifeinrichting 30 angepasst. Durch eine Bewegung des Außenschafts 70 in axialer Richtung und eine nachfolgende Rotation relativ zur Greifeinrichtung 30 können die Knaggen 37 durch die axialen Abschnitte 73 in die umfänglichen Abschnitte 74 eingeführt werden bis zu der in Figur 5 gezeigten Konfiguration. Bei der in Figur 5 gezeigten Konfiguration bzw. Anordnung der Knaggen 37 in den umfänglichen Abschnitten 74 der L-förmigen Schlitze am distalen Ende 72 des Außenschafts 70 sind der Außenschaft 70 und die Greifeinrichtung 30 hinsichtlich axialer Kräfte und Bewegungen miteinander (von Spiel abgesehen) starr gekoppelt.

Im Vergleich der Figuren 4 und 5 ist erkennbar, dass die umfänglichen Abschnitte 64, 74 der L-förmigen Schlitze an den distalen Enden 62, 72 des Innenschafts 60 einerseits und des Außenschafts 70 andererseits sich von den axialen Abschnitten 63, 73 aus in entgegengesetzten Richtungen erstrecken. Entsprechend ist die Richtung 92, in der der Außenschaft 70 relativ zur Greifeinrichtung 30 zu rotieren ist, um die in Figur 5 gezeigte gekoppelte Konfiguration zu erreichen, entgegengesetzt zu der Richtung 91, in der der Innenschaft 60 relativ zur Greifeinrichtung 30 und zur Schneideeinrichtung 50 zu rotieren ist, um die in Figur 4 gezeigte gekoppelte Konfiguration zu erreichen.

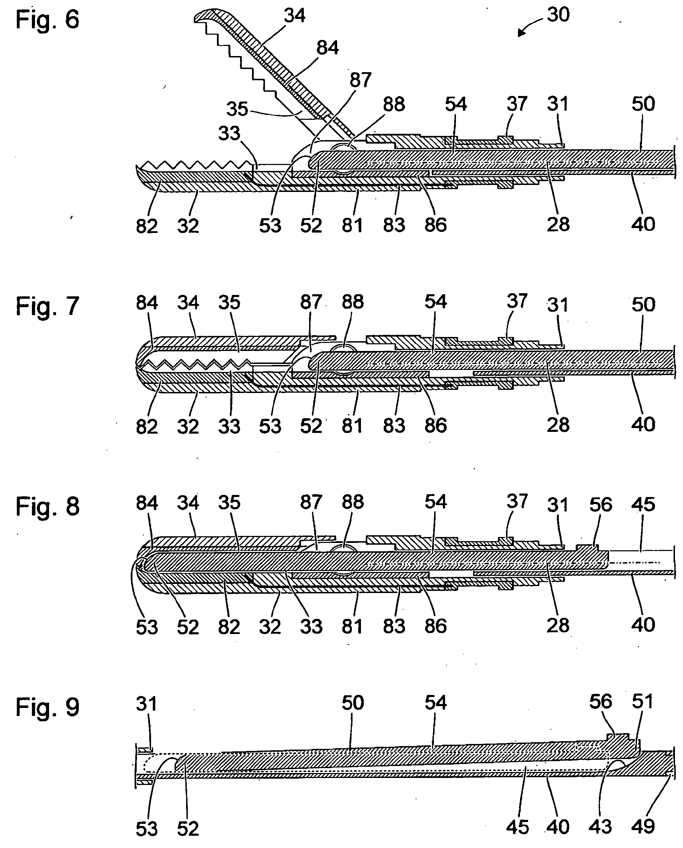

Figur 6 zeigt eine schematische Schnittdarstellung des Werkzeugs aus den Figuren 2, 4 und 5. Die darstellte Schnittebene enthält die Längsachse 28 (vgl. Figur 1).

Die Greifeinrichtung 30 umfasst einen Grundkörper 81, der ein elektrisch isolierendes Material aufweist und aus mehreren Elementen gefügt sein kann. Der Grundkörper 81 erstreckt sich vom proximalen Ende 31 der Greifeinrichtung 30 bis zu deren proximalem Ende und bildet insbesondere das feststehende Maulteil 32. Das feststehende Maulteil 32 umfasst einen Greifflächeneinsatz 82 aus Metall. Die Knaggen 37 sind Bestandteile eines hülsenförmigen metallischen Bestandteils, das durch einen zumindest teilweise im Grundkörper 81 der Greifeinrichtung 30 angeordneten Draht oder eine andere elektrisch leitfähige Verbindung 83 mit dem Greifflächeneinsatz 82 am feststehenden Maulteil 32 verbunden ist.

Das schwenkbare Maulteil 34 weist einen metallischen Greifflächeneinsatz 84 auf. Im Grundkörper 81 ist ein metallisches Lagerbauteil 86 angeordnet, das von den Knaggen 37 und dem Greifflächeneinsatz 82 des feststehenden Maulteils 32 durch den Grundkörper 81 elektrisch isoliert ist. Ein Achszapfen 88 ist mit dem schwenkbaren Maulteil 34 starr verbunden und um eine Schwenkachse senkrecht zur Schnittebene der Figur 6 rotierbar im Lagerbauteil 86 gelagert. Der Achszapfen 88 und ein parallel zur Schnittebene der Figur 6 angeordneter steg- oder plattenförmiger Bereich des Lagerbauteils 86 sind vom Betrachter aus gesehen hinter der Schnittebene der Figur 6 angeordnet. Symmetrisch zur Schnittebene der Figur 6 sind ein weiterer Achszapfen am schwenkbaren Maulteil 34 und ein weiterer plattenförmiger Bereich des Lagerbauteils 86, in dem der weitere Achszapfen gelagert ist, vor der Schnittebene der Figur 6 bzw. zwischen der Schnittebene und dem Betrachter angeordnet. Das Lagerbauteil 86 und die Achszapfen 88 definieren die senkrecht zur Schnittebene der Figur 6 angeordnete Schwenkachse des schwenkbaren Maulteils 34. Die Achszapfen 88 sind elektrisch leitfähig mit dem Greifflächeneinsatz 84 am schwenkbaren Maulteil 34 verbunden. Insbesondere sind die Achszapfen 88 mit je einem sich parallel zum schwenkbaren Maulteil 34 erstreckenden Holm einstückig ausgebildet, wobei die Holme und der Greifflächeneinsatz 84 geschweißt, gelötet, geschraubt oder anderweitig gefügt sind.

Die Übertragungsstange 40 ragt vom proximalen Ende 31 der Greifeinrichtung 30 her in den Grundkörper 81 hinein. Die Übertragungsstange 40 ist auf in Figur 6 nicht sichtbare Weise derart mit dem schwenkbaren Maulteil 34 mechanisch gekoppelt, dass eine translatorische Bewegung der Übertragungsstange 40 parallel zur Längsachse 28 mit einer Schwenkbewegung des schwenkbaren Maulteils 34 um die durch die Achszapfen 88 definierte Schwenkachse senkrecht zur Schnittebene der Figur 6 einhergeht. Bei der in Figur 6 gezeigten distalen Position der Übertragungsstange 40 nimmt das schwenkbare Maulteil 34 eine offene bzw. vom feststehenden Maulteil 32 beabstandete Position ein.

Die Übertragungsstange 40 aus einem metallischen Werkstoff ist elektrisch leitfähig mit den Achszapfen 88 und dem Greifflächeneinsatz 84 am schwenkbaren Maulteil 34 verbunden. Die Übertragungsstange 40 ist durch den Grundkörper 81 der Greifeinrichtung 30 von den Knaggen 37 und vom Greifflächeneinsatz 82 des feststehenden Maulteils 32 elektrisch isoliert.

Die Schneideeinrichtung 50 ist in einer Nut in der Übertragungsstange 40, die nur in den Figuren 8 und 9 mit einem Bezugszeichen versehen ist, und in einer Nut 87 im Lagerbauteil 86 angeordnet. Die Nut 87 im Lagerbauteil 86 ist in Richtung senkrecht zur Schnittebene der Figur 6 durch die erwähnten plattenförmigen Bereiche des Lagerbauteils 86, in denen die Achszapfen 88 des schwenkbaren Maulteils 34 gelagert sind, begrenzt bzw. wird durch den Zwischenraum zwischen den beiden plattenförmigen Bauteilen gebildet. In Verlängerung der Nut in der Übertragungsstange 40 und der Nut 87 im Lagerbauteil 86 nach distal, sind eine Nut 33 im feststehenden Maulteil 32, insbesondere im Greifflächeneinsatz 82 des feststehenden Maulteils 32, und eine Nut 35 im schwenkbaren Maulteil 34, insbesondere im Greifflächeneinsatz 84 des schwenkbaren Maulteils 34 vorgesehen.

In Figur 6 sind Querschnitte von elektrisch leitfähigen Bauteilen, insbesondere der Knaggen 37, der Übertragungsstange 40, der Schneideeinrichtung 50, der Greifflächeneinsätze 82, 84 und des Lagerbauteils 86, mit einer engen Schraffur dargestellt. Querschnitte von elektrisch isolierenden Bauteilen, insbesondere des Grundkörpers 81, sind mit weiten Schraffuren dargestellt. Wie bereits erwähnt, existieren eine elektrisch leitfähige Verbindung zwischen der Übertragungsstange 40 und dem Greifflächeneinsatz 84 am schwenkbaren Maulteil 34 über die Achszapfen 88 und die erwähnten Holme und eine elektrisch leitfähige Verbindung 83 zwischen den Knaggen 37 und dem Greifflächeneinsatz 82 am feststehenden Maulteil 32. Die Knaggen 37 und der Greifflächeneinsatz 84 am schwenkbaren Maulteil 34 sind von der Übertragungsstange 40 und dem Greifflächeneinsatz 82 am feststehenden Maulteil 32 elektrisch isoliert, solange die Greifflächeneinsätze 82, 84 einander nicht berühren. Somit ist die Greifeinrichtung 30 als bipolares elektrochirurgisches Instrument verwendbar, wobei ein Pol bzw. ein Potential über die Übertragungsstange 40 und der andere Pol bzw. das andere Potential über einen Außenschaft und die Knaggen 37 zugeführt wird.

Figur 7 zeigt eine weitere schematische Schnittdarstellung der Greifeinrichtung 30 aus den Figuren 2 und 4 bis 6. Die Schnittebene der Figur 7 entspricht der Schnitteben der Figur 6. In Figur 7 sind die Übertragungsstange 40 in einer proximalen Position und das schwenkbare Maulteil 34 in einer korrespondierenden geschlossenen bzw. am feststehenden Maulteil 32 anliegenden Position gezeigt. In Figur 7 nicht gezeigtes Gewebe zwischen den Maulteilen 32, 34 kann durch Stromfluss zwischen den Greifflächeneinsätzen 82, 84 koaguliert oder verödet werden.

In Figur 7 ist erkennbar, dass die Nuten 33, 35 in den Maulteilen 32, 34 eine Fortsetzung der Nut in der Übertragungsstange 40 und der Nut 87 im Lagerbauteil 86 nach distal bilden. Die Nut in der Übertragungsstange 40, die Nut 87 im Lagerbauteil 86 und die Nuten 33, 35 in den Maulteilen 32,34 bilden einen Kanal, in dem die Schneideeinrichtung 50 zwischen einer proximalen Position (insbesondere der in den Figuren 6 und 7 gezeigten Position) und einer distalen Position spiel- und reibungsarm geführt und bewegbar bzw. verschiebbar ist. Dazu weisen insbesondere die Nut in der Übertragungsstange 40, die Nut 87 im Lagerbauteil 86 und die Nuten 33, 35 in den Maulteilen 32, 34 Querschnitte (in Schnittebenen senkrecht zu den Schnittebenen der Figuren 6 und 7 und senkrecht zur Längsachse 28) auf, die zum Querschnitt der Schneideeinrichtung 50, insbesondere ihres stabförmigen Bereichs 54 korrespondieren bzw. nur wenig größer als dieser ausgebildet sind.

Figur 8 zeigt eine weitere schematische Schnittdarstellung der Greifeinrichtung 30 aus den Figuren 2 und 4 bis 7. Die Schnittebene der Figur 8 entspricht den Schnittebenen der Figuren 6 und 7. Wie in Figur 7 sind auch in Figur 8 die Übertragungsstange 40 in einer proximalen Position und das schwenkbare Maulteil 34 in einer geschlossenen Position gezeigt.

In Figur 8 ist die Schneideeinrichtung 50 in einer distalen Position gezeigt. Dadurch ist die Nut 45 in der Übertragungsstange 40 proximal der Schneideeinrichtung 50 sichtbar. In der in Figur 8 gezeigten distalen Position der Schneideeinrichtung 50 füllt diese die Nuten 33, 35 in den Maulteilen 32, 34 fast vollständig aus. Die Schneide 53 an der Schneideeinrichtung 50 befindet sich unmittelbar proximale der distalen Enden der Maulteile 32, 34. Bei der Bewegung der Schneideeinrichtung von der in Figur 7 gezeigten proximalen Position zu der in Figur 8 gezeigten distalen Position durchtrennt die Schneide 53 der Schneideeinrichtung 50 in den Figuren nicht dargestelltes Gewebe zwischen den Maulteilen 32, 34, insbesondere nach der Elektrokauterisation des Gewebes.

Am proximalen Ende 51 der Schneideeinrichtung 50 ist der auch in den Figuren 3 und 4 dargestellte Vorsprung 56 sichtbar, der mit dem distalen Ende 62 eines Innenschafts 60 (vgl. Figur 4) lösbar formschlüssig gekoppelt sein kann und eine Bewegung der Schneideeinrichtung 50 zwischen den in den Figuren 7 und 8 gezeigten Positionen mittels des Innenschafts 60 ermöglicht.

Figur 9 zeigt eine weitere schematische Schnittdarstellung, deren Schnittebene den Schnittebenen der Figuren 6 bis 8 entspricht. Der in Figur 9 gezeigte Ausschnitt ist gegenüber dem in den Figuren 6 bis 8 gezeigten Ausschnitt nach proximal verschoben. Am distalen (linken) Bildrand ist das proximale Ende 31 der Greifeinrichtung 30 (vgl. Figuren 6 bis 8) sichtbar. Am proximalen (rechten) Bildrand ist ein Isoliermantel 49 an der Übertragungsstange 40 sichtbar.

Die Nut 45 in der Übertragungsstange 40 weist an ihrem proximalen Ende einen rampenförmigen Bereich 43 mit einer zunehmenden Steigung auf. Im rampenförmigen Bereich 43 nimmt die Tiefe der Nut kontinuierlich bzw. stetig ab. Die Schneideeinrichtung 50 ist in Figur 9 zweimal dargestellt. Die lediglich durch gestrichelte Konturen dargestellte Position der Schneideeinrichtung 50 liegt weit proximal der in den Figuren 6 und 7 dargestellten Position, jedoch vom Vorsprung 56 abgesehen, vollständig innerhalb der Nut 45 in der Übertragungsstange 40. Bei einer Verschiebung der Schneideeinrichtung 50 noch weiter nach proximal nimmt die Schneideeinrichtung 50 die in Figur 9 in durchgezogenen Linien dargestellte Position ein. In dieser Position ist das proximale Ende 51 der Schneideeinrichtung 50 durch den rampenförmigen Bereich 43 am proximalen Ende der Nut 45 aus der Nut 45 teilweise herausgehoben.

Somit kann durch bloße Verschiebung der Schneideeinrichtung 50 (beispielsweise durch entsprechende Krafteinwirkung eines Fingers auf den Vorsprung 56) die Schneideeinrichtung 50 aus der Nut 45 herausgehoben und danach entnommen werden. Da dabei zunächst das proximale Ende 51 der Schneideeinrichtung 50 aus der Nut herausgehoben wird und die Schneideeinrichtung dann am proximalen Ende 51 gefasst werden kann, ist das Risiko einer Verletzung an der Schneide 53 gering.

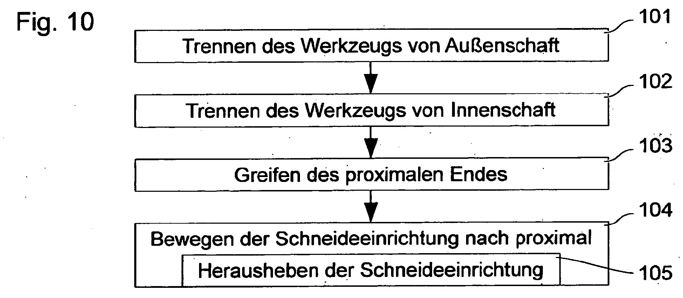

Figur 10 zeigt ein schematisches Flussdiagramm eines Verfahrens zum Zerlegen eines Werkzeugs für ein medizinisches Instrument. Das Verfahren ist insbesondere an einem Werkzeug mit den oben anhand der Figuren 1 bis 9 dargestellten Merkmalen ausführbar, kann jedoch auch auf Werkzeuge mit anderen Merkmalen angewendet werden. Zur Vereinfachung des Verständnisses werden nachfolgend beispielhaft Bezugszeichen aus den Figuren 1 bis 9 verwendet.

Bei einem ersten Schritt 101 wird das Werkzeug 30, 50 vom distalen Ende 72 eines Außenschafts 70 getrennt bzw. gelöst, insbesondere durch Lösen einer Bajonettkupplung. Bei einem zweiten Schritt 102 wird das Werkzeug 30, 50 von einem distalen Ende 62 eines Innenschafts 60 oder einer anderen Übertragungseinrichtung getrennt bzw. gelöst. Dabei wird insbesondere eine mechanische Verbindung zwischen dem distalen Ende 62 der Übertragungseinrichtung 60 und einer Schneideeinrichtung 50 gelöst. Bei einem dritten Schritt 103 wird das proximale Ende 51 der Schneideeinrichtung 50 ergriffen. Bei einem vierten Schritt 104 wird die Schneideeinrichtung 50 relativ zu anderen Bestandteilen des Werkzeugs nach proximal bewegt. Der dritte Schritt 103 und der vierte Schritt 104 werden insbesondere ausgeführt, indem ein Finger auf einen Vorsprung 56 an der Schneideeinrichtung 50 gelegt, gegen diesen gedrückt und mit dem Finger der Vorsprung 56 und die Schneideeinrichtung 50 nach proximal geschoben werden. Beim Bewegen 104 der Schneideeinrichtung 50 nach proximal wird bei einem fünften Schritt 105 die Schneideeinrichtung 50 aus einer Nut 45 herausgehoben. Dies geschieht insbesondere bei Erreichen des proximalen Endes der Nut 45.

### Bezugszeichen

- 10: medizinisches Instrument
- 11: proximales Ende des medizinischen Instruments 10
- 12: distales Ende des medizinischen Instruments 10
- 18: Handhabungseinrichtung am proximalen Ende 11 des medizinischen Instruments 10
- 19: Verriegelungseinrichtung an der Handhabungseinrichtung 18
- 20: Schaft des medizinischen Instruments 10
- 21: proximales Ende des Schafts 20
- 22: distales Ende des Schafts 20
- 28: Längsachse des Schafts 20
- 30: Greifeinrichtung am distalen Ende 12 des medizinischen Instruments 10
- 31: proximales Ende der Greifeinrichtung 30
- 32: feststehendes Maulteil der Greifeinrichtung 30
- 33: Nut im feststehenden Maulteil 32 der Greifeinrichtung 30
- 34: schwenkbares Maulteil der Greifeinrichtung 30
- 35: Nut im schwenkbaren Maulteil 34 der Greifeinrichtung 30
- 37: Knagge am proximalen Ende 31 der Greifeinrichtung 30
- 40: Übertragungsstange des medizinischen Instruments 10
- 43: rampenförmiger Bereich am proximalen Ende der Nut 45
- 45: Nut in der Übertragungsstange 40
- 49: Isoliermantel an der Übertragungsstange 40
- 50: Schneideeinrichtung am distalen Ende 12 des medizinischen Instruments 10
- 51: proximales Ende der Schneideeinrichtung 50
- 52: distales Ende der Schneideeinrichtung 50
- 53: Schneide an der Schneideeinrichtung 50
- 54: stabförmiger Bereich der Schneideeinrichtung 50
- 56: Vorsprung am proximalen Ende 51 der Schneideeinrichtung 50
- 60: Innenschaft des medizinischen Instruments 10
- 62: distales Ende des Innenschafts 60
- 63: axialer Abschnitt eines L-förmigen Schlitzes am distalen Ende 62
- 64: umfänglicher Abschnitt eines L-förmigen Schlitzes am distalen Ende 62
- 69: Isoliermantel am Innenschaft 60
- 70: Außenschaft des medizinischen Instruments 10
- 72: distales Ende des Außenschafts 70
- 73: axialer Abschnitt eines L-förmigen Schlitzes am distalen Ende 72
- 74: umfänglicher Abschnitt eines L-förmigen Schlitzes am distalen Ende 72
- 81: Grundkörper der Greifeinrichtung 30
- 82: Greifflächeneinsatz am feststehenden Maulteil 32
- 83: leitfähige Verbindung zwischen Knagge 37 und Greiffiächeneinsatz 82 am feststehenden Maulteil 32
- 84: Greifflächeneinsatz am schwenkbaren Maulteil 34
- 86: Lagerbauteil
- 87: Nut im Lagerbauteil 86
- 88: Achszapfen am schwenkbaren Maulteil 34
- 101: erster Schritt (Trennen des Werkzeugs von Außenschaft)
- 102: zweiter Schritt (Trennen des Werkzeugs von Innenschaft)
- 103: dritter Schritt (Greifen der Schneideeinrichtung)
- 104: vierter Schritt (Bewegen der Schneideeinrichtung nach proximal)
- 105: fünfter Schritt (Herausheben der Schneideeinrichtung)

## Patentansprüche

1. Werkzeug (30, 50) für ein medizinisches Instrument (10), mit:
einer Wirkeinrichtung (30);
einer Übertragungsstange (40), deren distales Ende mit der Wirkeinrichtung (30) gekoppelt ist, zum Übertragen zumindest entweder einer Kraft oder eines Drehmoments zu der Wirkeinrichtung (30);
einer Schneideeinrichtung (50), die zum Schneiden von Gewebe in einem Kanal (33, 35, 45) in der Übertragungsstange (40) und in der Wirkeinrichtung (30) bewegbar ist,
**dadurch gekennzeichnet, dass**
der Kanal eine Nut (45) in der Übertragungsstange (40) umfasst,
und die Nut (45) in einem Bereich (43) nahe dem proximalen Ende der Nut (45) eine nach proximal **kontinuierlich abnehmende Tiefe** aufweist.

2. Werkzeug (30, 50) nach dem vorangehenden Anspruch, bei dem die Schneideeinrichtung (50) ausgebildet ist, um bei einer Bewegung in Richtung **parallel** zur Längsachse (28) des Werkzeugs (30, 50) zu **schneiden.**

3. Werkzeug (30, 50) nach einem der vorangehenden Ansprüche, bei dem die Nut (45) so ausgebildet ist, dass eine Bewegung der Schneideeinrichtung (50) **nach proximal** mit einem **Herausheben** der Schneideeinrichtung (50) aus der Nut (45) einher geht.

4. Werkzeug (30, 50) nach einem der vorangehenden Ansprüche, ferner mit:
einer **Kupplungseinrichtung** (37) zum lösbaren mechanischen Verbinden des Werkzeugs (30, 50) mit einem distalen Ende (72) eines Außenschafts (70),
wobei in allen für die Schneideeinrichtung (50) während der Verwendung des Werkzeugs (30, 50) vorgesehenen Positionen das proximale Ende (51) der Schneideeinrichtung (50) proximal der Kupplungseinrichtung (37) und das distale Ende (52) der Schneideeinrichtung (50) distal der Kupplungseinrichtung (37) angeordnet sind.

5. Werkzeug (30, 50) nach einem der vorangehenden Ansprüche, bei dem die Schneideeinrichtung (50) zwischen ihrem proximalen Ende (51) und ihrem distalen Ende (52) einen **stabförmigen** Bereich (54) aufweist.

6. Werkzeug (30, 50) nach einem der vorangehenden Ansprüche, bei dem die Schneideeinrichtung (50) einen **Vorsprung** (56), der in einer Richtung senkrecht zur vorgesehenen Bewegungsrichtung der Schneideeinrichtung (50) vorsteht, aufweist.

7. Werkzeug (30, 50) nach dem vorangehenden Anspruch, bei dem die Schneideeinrichtung (50) in einer während der Verwendung des Werkzeugs (30, 50) vorgesehenen Position von dem Vorsprung (56) abgesehen **vollständig in dem Kanal** (35, 45) angeordnet ist.

8. **Medizinisches Instrument** (10) mit:
einem Werkzeug (30, 50) nach einem der vorangehenden Ansprüche;
einem **Innenschaft** (60), dessen distales Ende (62) mit der Schneideeinrichtung (50) lösbar mechanisch verbindbar ist.

9. Medizinisches Instrument (10) nach dem vorangehenden Anspruch in Rückbezug auf Anspruch 6, bei dem der Innenschaft (60) an seinem distalen Ende (62) einen **L-förmigen Schlitz** (63, 64) oder eine L-förmige Nut zur Aufnahme des Vorsprungs (56) an der Schneideeinrichtung (50) umfasst.

10. **Verfahren zum Zerlegen eines Werkzeugs** (30, 50) für ein medizinisches Instrument (10), wobei das Werkzeug eine **Wirkeinrichtung** (30) und eine **Schneideeinrichtung** (50) in einem Kanal (33, 35,45) umfasst, mit folgenden Schritten:
**Trennen** (102) einer mechanischen Verbindung zwischen einem distalen Ende (62) einer Übertragungseinrichtung (60) und der Schneideeinrichtung (50);
**Bewegen** (104) der Schneideeinrichtung (50) relativ zum Werkzeug (30) **nach proximal,** wobei die Schneideeinrichtung (50) aus dem Kanal (33, 35, 45) herausgehoben (105) wird,
**dadurch gekennzeichnet, dass**
das proximale Ende der Schneideeinrichtung (50) an einem rampenförmigen Bereich (43) am proximalen Ende einer Nut (45) in einer Übertragungsstange (40) gleitet.

11. Verfahren nach dem vorangehenden Anspruch, ferner mit folgendem Schritt:
**Trennen** (101) eines distalen Endes (72) eines Außenschafts (70) von dem Werkzeug (30, 50) vor dem Trennen (102) der mechanischen Verbindung zwischen dem distalen Ende (62) des Innenschafts (60) und der Schneideeinrichtung (50).

## Claims

1. Tool (30, 50) for a medical instrument (10), comprising:
an effecting device (30);
a transmission rod (40), of which the distal end is coupled to the effecting device (30), for transmitting at least either a force or a torque to the effecting device (30);
a cutting device (50), which, to cut tissue, is movable in a channel (33, 35, 45) in the transmission rod (40) and in the effecting device (30),
**characterized in that** the channel comprises a groove (45) in the transmission rod (40),
and the groove (45) has a proximally continuously reducing depth in a region (43) close to the proximal end of the groove (45).

2. Tool (30, 50) according to the preceding claim, wherein the cutting device (50) is designed to cut with a movement in the direction parallel to the longitudinal axis (28) of the tool (30, 50).

3. Tool (30, 50) according to one of the preceding claims, wherein
the groove (45) is designed such that a movement of the cutting device (50) in proximal direction is accompanied by a lifting out of the cutting device (50) from the groove (45).

4. Tool (30, 50) according to one of the preceding claims, further comprising:
a coupling device (37) for releasable mechanical connection of the tool (30, 50) to a distal end (72) of an outer shaft (70),
wherein, in all positions provided for the cutting device (50) during the use of the tool (30, 50), the proximal end (51) of the cutting device (50) is arranged proximally of the coupling device (37) and the distal end (52) of the cutting device (50) is arranged distally of the coupling device (37).

5. Tool (30, 50) according to one of the preceding claims, wherein the cutting device (50) comprises a bar-shaped region (54) between its proximal end (51) and its distal end (52).

6. Tool (30, 50) according to one of the preceding claims, wherein the cutting device (50) comprises a protrusion (56), which protrudes in a direction perpendicular with respect to the provided direction of movement of the cutting device (50).

7. Tool (30, 50) according to the preceding claim, wherein the cutting device (50) is arranged completely in the channel (35, 45), apart from the protrusion (56), in a position provided during the use of the tool (30, 50).

8. Medical instrument (10), comprising:
a tool (30, 50) according to one of the preceding claims;
an inner shaft (60), of which the distal end (62) is releasably mechanically connectable to the cutting device (50).

9. Medical instrument (10) according to the preceding claim with reference to Claim 6, wherein the inner shaft (60) at its distal end (62) comprises an L-shaped slit (63, 64) or an L-shaped groove for receiving the protrusion (56) on the cutting device (50).

10. Method for disassembling a tool (30, 50) for a medical instrument (10), wherein the tool comprises an effecting device (30) and a cutting device (50) in a channel (33, 35, 45), said method comprising the following steps:
separating (102) a mechanical connection between a distal end (62) of a transfer device (60) and the cutting device (50);
moving (104) the cutting device (50) relative to the tool (30) in proximal direction, wherein the cutting device (50) is lifted out (105) from the channel (33, 35, 45),
**characterized in that**
the proximal end of the cutting device (50) slides over a ramp-shaped region (43) at the proximal end of a groove (45) in a transmission rod (40).

11. Method according to the preceding claim, further comprising the following step:
separating (101) a distal end (72) of an outer shaft (70) from the tool (30, 50) before separating (102) the mechanical connection between the distal end (62) of the inner shaft (60) and the cutting device (50).

## Revendications

1. Outil (30, 50) pour un instrument médical (10), comprenant :
un dispositif fonctionnel (30) ;
une tige de transfert (40) dont l'extrémité distale est accouplée au dispositif fonctionnel (30) pour transférer au moins soit une force soit un couple au dispositif fonctionnel (30) ;
un dispositif de coupe (50) qui peut être déplacé pour découper des tissus dans un canal (33, 35, 45) dans la tige de transfert (40) et dans le dispositif fonctionnel (30),
**caractérisé en ce que**
le canal comprend une rainure (45) dans la tige de transfert (40),
et la rainure (45) présente dans une région (43) à proximité de l'extrémité proximale de la rainure (45) une profondeur diminuant en continu dans la direction proximale.

2. Outil (30, 50) selon la revendication précédente, dans lequel le dispositif de coupe (50) est réalisé de manière à découper dans le cas d'un déplacement dans la direction parallèle à l'axe longitudinal (28) de l'outil (30, 50).

3. Outil (30, 50) selon l'une quelconque des revendications précédentes, dans lequel la rainure (45) est réalisée de telle sorte qu'un déplacement du dispositif de coupe (50) dans la direction proximale soit associé à un soulèvement du dispositif de coupe (50) hors de la rainure (45).

4. Outil (30, 50) selon l'une quelconque des revendications précédentes, comprenant en outre :
un dispositif d'accouplement (37) pour la connexion mécanique amovible de l'outil (30, 50) à une extrémité distale (72) d'une tige extérieure (70),
dans toutes les positions prévues pour le dispositif de coupe (50) pendant l'utilisation de l'outil (30, 50), l'extrémité proximale (51) du dispositif de coupe (50) étant disposée proximalement au dispositif d'accouplement (37) et l'extrémité distale (52) du dispositif de coupe (50) étant disposée distalement au dispositif d'accouplement (37).

5. Outil (30, 50) selon l'une quelconque des revendications précédentes, dans lequel le dispositif de coupe (50) présente entre son extrémité proximale (51) et son extrémité distale (52) une région en forme de barre (54).

6. Outil (30, 50) selon l'une quelconque des revendications précédentes, dans lequel le dispositif de coupe (50) présente une saillie (56) qui fait saillie dans une direction perpendiculaire à la direction de déplacement prévue du dispositif de coupe (50).

7. Outil (30, 50) selon la revendication précédente, dans lequel le dispositif de coupe (50) est disposé dans une position prévue pendant l'utilisation de l'outil (30, 50) complètement à l'intérieur du canal (35, 45) à l'exception de la saillie (56).

8. Instrument médical (10) comprenant :
un outil (30, 50) selon l'une quelconque des revendications précédentes ;
une tige intérieure (60) dont l'extrémité distale (62) peut être connectée mécaniquement de manière amovible au dispositif de coupe (50).

9. Instrument médical (10) selon la revendication précédente lorsqu'elle se rapporte à la revendication 6, dans lequel la tige intérieure (60) comprend au niveau de son extrémité distale (62) une fente en forme de L (63, 64) ou une rainure en forme de L pour recevoir la saillie (56) au niveau du dispositif de coupe (50).

10. Procédé pour désassembler un outil (30, 50) pour un instrument médical (10), l'outil comprenant un dispositif fonctionnel (30) et un dispositif de coupe (50) dans un canal (33, 35, 45), comprenant les étapes suivantes :
déconnecter (102) une connexion mécanique entre une extrémité distale (62) d'un dispositif de transfert (60) et le dispositif de coupe (50) ;
déplacer (104) le dispositif de coupe (50) par rapport à l'outil (30) dans la direction proximale, le dispositif de coupe (50) étant soulevé (105) hors du canal (33, 35, 45),
**caractérisé en ce que**
l'extrémité proximale du dispositif de coupe (50) glisse au niveau d'une région en forme de rampe (43) sur l'extrémité proximale d'une rainure (45) dans une tige de transfert (40).

11. Procédé selon la revendication précédente, comprenant en outre l'étape suivante :
séparation (101) d'une extrémité distale (72) d'une tige extérieure (70) et de l'outil (30, 50) avant la déconnexion (102) de la connexion mécanique entre l'extrémité distale (62) de la tige intérieure (60) et du dispositif de coupe (50).
